# EUROPEAN PATENT APPLICATION

(11) **EP 2 468 262 A1**
(43) Date of publication of application: **27.06.2012**
(21) Application number: 10193880.1
(22) Date of filing: 06.12.2010
(51) Int. Cl.: A61K 9/20, A61K 9/48, A61K 9/50, A61K 45/06

(54) **Pharmaceutical composition comprising dutasteride**

(71) Applicant: Krka Tovarna Zdravil, D.D., Novo Mesto, 8501 Novo Mesto (SI)
(72) Inventor: Segula Zakelj, Mojca, SLO-1000 Ljubljana (SI); Kroselj, Vesna, SLO-8000 Novo mesto (SI); Vrecer, Franc, SLO-8351 Straza pri Novem mestu (SI); Rustomjee, Maharukh T., 400007 Mumbai (IN); Yadav, Kamala S., 400058 Mumbai (IN); Gandhi, Anilkumar S., 400092 Mumbai (IN); Kulkarni, Sanjivani A., 400054 Mumbai (IN); Jadhav, Simta S., 421201 Thane (IN)
(74) Representative: UEXKÜLL & STOLBERG

(57) **Abstract**

The invention relates to a pharmaceutical composition comprising (a) at least one active ingredient selected from dutasteride or a pharmaceutically acceptable salt or solvate thereof and (b) a solubilizer.

## Description

The present invention relates to a pharmaceutical composition comprising dutasteride or a pharmaceutically acceptable salt or solvate thereof, a process for preparing such a composition as well as a pharmaceutical composition comprising dutasteride and at least one other active ingredient selected from alpha-1-adrenoceptors or antiandrogens.

Dutasteride is the generic name of (5α,17β)-N-{2,5 bis(trifluoromethyl)phenyl}-3-oxo-4-azaandrost-1-ene-17-carboxamide having the molecular formula C₂₇H₃₀F₆N₂O₂, and a molecular weight of 528.53 g/mol. As a synthetic 4-azasteroid compound it selectively inhibits type 1 and type 2 isoforms of 5α-reductase. This enzyme converts testosterone to dihydrotestosterone which is the androgen primarily responsible for the initial development and subsequent enlargement of the prostate gland. Therefore, dutasteride is primarily used for the treatment of moderate to severe symptoms of benign prostatic hyperplasia (BPH).

Dutasteride is marketed as Avodart^{®} soft gelatine capsules which contain 0.5 mg of dutasteride dissolved in a mixture of mono- and diglycerides of caprylic and capric acid as well as butylated hydroxytoluene. The inactive excipients of the capsule shell include gelatine, glycerin and yellow iron oxide. However, soft gelatine capsules like Avodart^{®} require special production equipment. Moreover, they can be difficult to be handled in case of leakage in the capsule shell.

Dutasteride is practically insoluble in water with reported solubility in water below the quantification limit of the assay (0.38 ng/ml). Hence, the bioavailability of dutasteride is related to its poor water solubility and may be improved by micronization of dutasteride and/or by dissolving dutasteride in solvents like oils.

Accordingly, EP 2 050 436 describes a pharmaceutical composition of dutasteride, wherein due to micronization the average particle size of dutasteride ranges from 2 µm to 10 µm to achieve dissolution of more than 80% of dutasteride in a test medium within 10 minutes.

However, due to dust formation micronization of dutasteride is very hazardous for employees involved. Dutasteride is a very potent and harmful molecule. Excessive exposure can damage the male sex glands, impair the fertility and have a teratogenic effect to unborn male children. Furthermore, it can also lead to impotence, decreased libido and ejaculation disorders. In view of the readily absorption of dutasteride through the skin and its very long elimination time, it is of particular importance to keep the handling with a micronized dutasteride material to a minimum.

IPCOM000154719D describes a pharmaceutical composition comprising dutasteride and alfuzosin which is an alpha-1-adrenergic receptor antagonist.

WO 2010/092596 describes an oral pharmaceutical composition of dutasteride comprising one or more surfactant(s), one or more co-surfactant(s) and one or more oil(s). Upon dilution with aqueous medium the composition forms a microemulsion with at least 95% of the particles having mean particle sizes below 200 nm. Likewise, IPCOM000173742D and KR 2010-16546 describe a self microemulsifying drug delivery system (SMEDDS) and/or a self-emulsifying lipidic formulation (SEDDS). However, these formulations and in particular the SMEDDS (Self Microemulsifying Drug Delivery System) described in WO 2010/092596 have several drawbacks. For example, relatively high concentrations of surfactant(s) and co- surfactant(s) are required. Surfactant(s) and co-surfactant(s) in high concentrations can modify the delivery of dutasteride in the gastrointestinal tract and the permeability of the active ingredient through the enterocytes. Moreover, the preparation of the composition includes several process steps. In addition, the composition exhibits stability problems with respect to hygroscopicity and sensitivity to oxygen.

Consequently, there is still a need for a pharmaceutical composition of dutasteride or a salt or solvate thereof which avoids the above mentioned disadvantages of the compositions of the prior art. In particular, a safe pharmaceutical composition of dutasteride exhibiting a good stability and providing a good availability of the active ingredient would be desirable. Preferably, the composition should be obtainable by a simple and quick process.

These objects are surprisingly solved by the pharmaceutical composition according to any one of claims 1 to 11 and 15. The invention also relates to the process according to any one of claims 12 to 14.

The invention is directed to a pharmaceutical composition which comprises
(a) at least one active ingredient selected from dutasteride or a pharmaceutically acceptable salt or solvate thereof and
(b) a solubilizer.

Preferably, the composition is in the form of a solid pharmaceutical composition. Furthermore, it is preferred that the composition is in a form for oral administration. In particular, the composition is in the form of tablets or capsules and preferably in the form of film coated tablets.

In one embodiment, the composition according to the invention comprises granules which comprise the active ingredient (a) and the solubilizer (b). Preferably, the granules comprise all of the active ingredient (a) and solubilizer (b) contained in the composition.

The component (a) of the composition according to the invention is an active ingredient selected from dutasteride or a pharmaceutically acceptable salt or solvate thereof. Preferably, the active ingredient (a) is free dutasteride.

The active ingredient (a) can be in a non-crystalline, i.e. amorphous, form or a crystalline form including any polymorphic or pseudopolymorphic forms such as solvates. For example, the polymorphic form obtained according to EP 0 719 718, polymorphic forms I or II or the amorphous form as described in US 7,022,854 or the crystalline form III as described in WO 2009/083258 can be used. Preferably, crystalline form I is used.

The impurity profile of dutasteride used in the present invention is according to the ICH guidelines (cf. Pharmazie, 62 (2007), p. 743-746; and WO 2007/120263).

Processes for preparing dutasteride or a pharmaceutically acceptable salt or solvate thereof are known and for example described in WO 95/07927 and US 5,565,467.

The particle size of dutasteride used in the present invention is not particularly limited and generally all particle sizes can be used. In particular, the particle size is not restricted to rather small particle sizes having an average particle size of 10 µm or less. In one embodiment, the d₉₀ particle size of active ingredient (a) can be more than 20 µm. It is preferred that the d₉₀ particle size of active ingredient (a) is less than 500 µm, preferably less than 250 µm and most preferably less than 150 µm.

It is preferred that the composition comprises 0.02 to 1 wt.-%, more preferably 0.05 to 0.7 wt.-% and most preferably 0.08 to 0.5 wt.-% of active ingredient (a).

It is also preferred that a single dosage form of the pharmaceutical composition according to the invention, such as a tablet or capsule, comprises 0.05 to 5 mg, preferably 0.1 to 1 mg and most preferably 0.3 and 0.8 mg like about 0.5 mg of active ingredient (a).

The component (b) of the composition according to the invention is a solubilizer. As used herein, the term "solubilizer" refers to a substance capable of improving the solubility and/or dissolution rate of active ingredient (a) in aqueous and in particular physiological medium.

In particular, the solubilizer has a low melting or softening point. As used herein, the term "melting point" refers to the temperature at which a substance completely transforms into a liquid state. Further, "softening point" refers to the temperature at which a substance transforms from solid into a waxy state. The softening point can be determined by thermomicroscopy or differential scanning method (DSC) such as defined in ISO 11357-3. In one embodiment, the solubilizer (b) is liquid at room temperature or at least at 60°C. Preferably, solubilizer (b) has a melting or softening point of less than about 170°C, more preferably less than about 150°C and most preferably less than about 100°C. In one embodiment, if solubilizer (b) is a polymeric solubilizer, a plasticizer can be included to decrease the softening point of the polymeric solubilizer.

Preferably, solubilizer (b) has a low HLB (hydrophilic-lipophilic balance) value based on the HLB system according to W.C. Griffin which is well known to those skilled in the art. In particular, solubilizer (b) has an HLB value of 1 to 7.5, preferably 1.5 to 7 and more preferably 2 to 6.5. If solubilizer (b) comprises a mixture of two or more solubilizers compounds, then it is preferred that each solubilizer compound has an HLB value of 1 to 7.5, preferably 1.5 to 7 and more preferably 2 to 6.5.

Preferably, solubilizer (b) is selected from the group consisting of propylene glycol esters, medium chain triglycerides, medium chain mono- and diglycerides, long chain mono- and diglycerides, propylene glycol, polyethylene glycols, block copolymers of polyethylene glycol and polypropylene glycol, sucrose fatty acid esters and mixtures thereof. More preferably, solubilizer (b) is selected from the group consisting of propylene glycol esters, medium chain triglycerides, medium chain mono- and diglycerides and long chain mono- and diglycerides. As used herein, "medium chain" refers to C₆-C₁₂ fatty acid esters of glycerol. "Long chain" refers to fatty acid esters of glycerol having more than 12 carbon atoms in the chain. Examples of suitable propylene glycol esters include propylene glycol caprylate (usually commercially available under Capryol^{®} PGMC), propylene glycol monocaprylate (usually commercially available under Capryol^{®} 90), propylene glycol dicaprylocaprate (usually commercially available under Captex^{®} 200 P), propylene glycol monolaurate, propylene glycol laurate, PEG 300 oleic glycerides and PEG 300 linoleic glycerides. Examples of suitable medium chain triglycerides include triglycerides of caprylic and capric fatty acids (Miglyol^{®}) and triglycerides of caprylic/capric acid (usually commercially available under Labrafac^{®} Lipophile WL 1349). Examples of suitable medium chain mono-and diglycerides include glyceryl caprylate/caprate. Examples of suitable long chain mono- and diglycerides include glyceryl monooleate and caprylocaproyl macrogolglycerides. Examples of suitable polyethylene glycols include polyethylene glycol 400. Examples of suitable block copolymers of polyethylene glycol and polypropylene glycol include polaxamer 188 and polaxamer 407.

It is preferred that solubilizer (b) is a propylene glycol ester like propylene glycol caprylate, propylene glycol monocaprylate, propylene glycol dicaprylocaprate or a mixture thereof. Preferably, solubilizer (b) is propylene glycol caprylate or propylene glycol monocaprylate. It is particularly preferred that solubilizer (b) is propylene glycol monocaprylate.

The composition preferably comprises 1 to 90 wt.-%, more preferably 3 to 80 wt.-% and most preferably 5 to 75 wt.-% like 5 to 25 wt.-%, 8 to 25 wt.-% or 11 to 20 wt.-% of solubilizer (b).

Preferably, in addition to solubilizer (b) the composition does not comprise a further surfactant. If the composition comprises granules comprising active ingredient (a) and solubilizer (b), then the granules do preferably not comprise a further surfactant.

In particular, the pharmaceutical composition according to the invention and/or the granules thereof do not comprise a further surfactant having an HLB value of 8 or more based on the HLB system according to W.C. Griffin which is well known to those skilled in the art.

The composition and/or the granules thereof do particularly not comprise a surfactant selected from the group consisting of PEG 400 monostearate, polyoxy 8 stearate, PEG 660 hydroxystearate, Macrogol 15 hydroxystearate, polyoxyl 10 oleyl ether, oleth 10 PEG monooleyl ether, polysorbate 80, polysorbate 85, polyoxyl 35 castor oil, polyoxyethyleneglycerol triricinoleate 35, polyoxyl-40-hydrogenated castor oil, caprylcaproyl polyoxyglycerides, hydrogenated palm kernel oil PEG-32 esters, lauryl polyoxyglycerides, lauryl macrogol glycerides and polyglyceryl-6-dioleate.

Naturally, the features described immediately above, i.e. the absence of a further surfactant and/or the absence of a surfactant having a specific HLB value and/or the absence of a surfactant selected from a specific group, can also apply to all preferred embodiments described herein, in particular all preferred composition embodiments, all preferred granule embodiments and all preferred process embodiments described herein.

In addition to components (a) and (b), the composition according to the invention can comprise at least one other excipient selected from the group consisting of inert adsorbents, diluents, binders, disintegrants, lubricants, antioxidants and combinations thereof.

It is particularly preferred that the composition comprises at least one inert adsorbent. Typically, the inert adsorbent has a high porosity and/or a high specific surface area, such as specific surface area of more than 25 m²/g. Examples of suitable inert adsorbents are selected from the group consisting of colloidal silicon dioxide, granulated colloidal silicon dioxide, porous silica gel (such as Sylysia^{®} sold by Fuji Chemicals), calcium silicate, aluminum magnesium silicate, porous silicates with specific surface areas of at least 25 m²/g, microcrystalline cellulose, lactose monohydrate, mannitol and mixtures thereof.

Preferably, the composition comprises 0 to 90 wt.-%, preferably 20 to 80, more preferably 40 to 75 wt.-% and most preferably 50 to 65 wt.-% of inert adsorbent.

The composition according to the invention can also comprise a diluent/filler. Preferably, the diluent is selected from pharmaceutically acceptable substances which are soluble in water at room temperature such as mono-, di-, oligo or polysaccharides such as lactose, sucrose, sugar alcohols like mannitol, xylitol, sorbitol, and dextrin; or pharmaceutically acceptable substances which are insoluble in water at room temperature, i.e. having a water-solubility of less than 0.2 g/ml, such as microcrystalline cellulose, calcium salts of phosphoric acid and the like.

Examples of binders which can suitably be used in the composition according to the present invention include, but are not restricted to water-soluble polymers selected from cellulose ether derivatives such as hydroxypropyl methylcellulose, hydroxypropyl cellulose, hydroxyethyl cellulose, methylcellulose, povidone, copovidone, polyvinyl alcohol-polyethylene glycol graft copolymer, polyvinyl alcohol or the like. Preferably, hydroxypropyl cellulose can be used as binder.

Further, the composition according to the invention can comprise a disintegrant, such as a disintegrant selected from croscarmellose sodium, crospovidone, sodium starch glycolate and the like. Preferably, croscarmellose sodium can be used as disintegrant.

Examples of lubricants useful in the composition according to the invention include calcium silicate, magnesium stearate, sodium stearyl fumarate, stearinic acid and colloidal silicon dioxide.

Although the composition according to the invention shows a satisfactory stability, it can comprise an antioxidant. The antioxidant can be selected from the group consisting of butylated hydroxytoluene (BHT), butylated hydroxyanisole (BHA), propyl galate, α-tocopherol, ascorbic acid, sodium ascorbate, ascorbyl palmitate, citric acid, EDTA and the like.

Optionally, the composition according to the invention comprises at least one coating. Preferably, the coating comprises a polymer selected from the group consisting of water insoluble or soluble cellulose derivatives such as methylcellulose, hydroxypropyl methylcellulose or ethylcellulose, polyvinyl acetate, methacrylic ester copolymers, ammonio methacrylate copolymers, aminoalkyl methacrylate copolymers, methacrylic acid copolymers, polyvinyl alcohol, polyethylene glycol and mixtures thereof.

Further, the coating can comprise at least one other excipient selected from the group consisting of plasticizers, antitacking agents, release rate modifiers, colorants, opacifiers, pigments, vehicles and mixtures thereof.

Preferably, the composition according to the invention is in the form of cores or tablets which can be coated with an appropriate material used for film coating, e.g. as described in Pharmaceutical Coating Technology (G. Cole (ed.), 1995). Film coating formulations usually contain components like polymer(s), plasticizer(s), colorant(s)/opacifier(s), vehicle(s). In film coating suspension the minor quantities of flavors, surfactants and waxes can be used. Film coating suspensions can also be used as ready-to-make preparations which are available on the market.

In a specific embodiment of the present invention, a tablet can be coated by a controlled release film coating comprising controlled release polymer which can be selected from water insoluble cellulose derivatives such as ethylcellulose, polyvinyl acetate, methacrylic ester copolymers (Eudragit NE) and ammonio methacrylate copolymers. Controlled release coating can further contain plasticizers which lower the glass transition temperature of controlled release polymers below 80°C, preferably below 60°C, antitacking agents such as talc, glycerol monostearate, release rate modifiers which can be selected from pharmaceutically acceptable substances having solubility in water at least 10 weight/vol-% such as mannitol, sorbitol, lactose, polyethylene glycol 1500, polyethylene glycol 6000, polyethylene glycol 20000, povidone, low viscosity cellulose ether derivatives such as hyprollose, hypromellose, hydroxyethyl cellulose, having a viscosity of 2% solution in water at room temperature 1 to 30 cp, preferably 2 to 15 cp, more preferably 3 to 9 cp determined according to the method described in US Pharmacopoeia, colorants and pigments such as titanium dioxide, iron oxides.

In another embodiment of the present invention, a tablet can be coated by a film coating having low permeability for water vapor and/or oxygen which comprises polymers such as aminoalkyl methacrylate copolymers (Eudragit E types), methacrylic acid copolymers, polyvinyl alcohol, methylcellulose, hypromellose, plasticizers such as polyethylene glycol having a molecular weight below 10,000, propylene glycol triethylcitrate, antitacking agents such as talc, glycerol monostearate, colorants and pigments such as titanium dioxide, iron oxides.

Furthermore, the invention relates to a pharmaceutical composition according to the invention which further comprises at least one second active ingredient selected from the group consisting of alpha-1-adrenoceptors, antiandrogens and pharmaceutically acceptable salts and solvates thereof.

Examples of suitable alpha-1-adrenoceptors include tamsulosin, silodosin, doxazosin, terazosin and alfuzosin. Bicalutamide is an example of a suitable antiandrogen. Thus, the composition according to the invention can further comprise at least one second active ingredient selected from the group consisting of tamsulosin, silodosin, doxazosin, terazosin, alfuzosin, bicalutamide and pharmaceutically acceptable salts and solvates thereof. In particular, the composition according to the invention can further comprise tamsulosin or a tamsulosin or a salt thereof like its hydrochloride salt.

Tamsulosin is the generic name of 5-[(2R)-2-[[2-(2-Ethoxyphenoxy)ethyl]amino]propyl]-2-methoxybenzenesulfonamide and is known as an α₁-adrenergic receptor antagonist. For many years, tamsulosin or a salt thereof and in particular its hydrochloride salt has been used in the treatment of benign prostatic hyperplasia (BPH).

In a preferred embodiment, the composition comprises a solid pharmaceutical composition of dutasteride according to the present invention combined with a solid composition containing the second active ingredient which preferably is tamsulosin or a salt thereof like its hydrochloride salt.

Accordingly, the present invention also relates to a pharmaceutical combination composition, comprising (a') a solid pharmaceutical composition of dutasteride according to the present invention and (b') a solid composition of the second active ingredient or a salt thereof like tamsulosin hydrochloride. Preferably, the combination composition is in the form of capsules, and in particular hard gelatine capsules, comprising (a') and (b').

The solid composition of dutasteride can be in the form of granules, pellets, tablets, microtablets, hard gelatin capsules or soft gelatin capsules. The solid composition containing the second active ingredient can be in the form of granules, pellets, microtablets or tablets. Both compositions are combined into a solid dosage form, e.g. by filling both compositions into hard capsules made of e.g. gelatin or hypromellose. Tablets containing both active substances like bilayered tablets, inlay tablets, i.e. a tablet containing one of both API represent a core around which is placed a second layer containing second API or tablets containing pellets can also be used as a combination composition. Inlay tablets can be produced by compression coating or conventional coating of a tablet core containing one API with a coating containing the second API.

Preferably, dutasteride in the form of film-coated tablets or granules according to the present invention and tamsulosin hydrochloride in the form of pellets, granules or tablets are filled together into a capsule. Most preferably, they are filled into hard gelatine capsules.

In the following preferred embodiments of the composition according to the invention are described. Preferably, the components of these embodiments can independently have the preferred meanings and properties described above.

According to a preferred embodiment, the composition according to the invention comprises
(a) at least one active ingredient selected from dutasteride or a pharmaceutically acceptable salt or solvate thereof,
(b) a solubilizer and
(c) at least one inert adsorbent.

According to another preferred embodiment, the composition according to the invention comprises
(a) dutasteride,
(b) a propylene glycol ester and
(c) at least one inert adsorbent.

According to another preferred embodiment, the composition according to the invention comprises
(a) dutasteride,
(b) propylene glycol monocaprylate and
(c) at least one inert adsorbent.

According to another preferred embodiment, the composition according to the invention comprises, based on the total weight of the composition,
(a) 0.08 to 0.5 wt.-% of dutasteride,
(b) 5 to 25 wt.-% of a propylene glycol ester like propylene glycol monocaprylate and
(c) 50 to 70 wt.-% of an inert adsorbent.

According to another preferred embodiment, the composition according to the invention essentially consists of
(a) at least one active ingredient selected from dutasteride or a pharmaceutically acceptable salt or solvate thereof,
(b) a solubilizer,
(c) optionally at least one inert adsorbent,
(d) optionally at least one other excipient selected from the group consisting of diluents, binders, disintegrants, lubricants, antioxidants and combinations thereof and
(f) optionally at least one coating.

The composition of this embodiment can optionally be filled into capsules.

According to another preferred embodiment, the composition according to the invention essentially consists of, based on the total weight of the composition,
(a) 0.08 to 0.5 wt.-% of dutasteride,
(b) 5 to 75 wt.-% of a solubilizer like a propylene glycol ester such as propylene glycol monocaprylate,
(c) 0 to 70 wt.-% of at least one inert adsorbent,
(d) optionally at least one other excipient selected from the group consisting of diluents, binders, disintegrants, lubricants, antioxidants and combinations thereof and
(f) optionally at least one coating.

The composition of this embodiment can optionally be filled into capsules.

As mentioned above, the pharmaceutical composition according to the invention can comprise granules which comprise the active ingredient (a) and the solubilizer (b).

According to a preferred embodiment, the granules comprise or essentially consist of
(a) at least one active ingredient selected from dutasteride or a pharmaceutically acceptable salt or solvate thereof,
(b) a solubilizer ,
(c) optionally at least one inert adsorbent,
(d) optionally at least one binder,
(e) optionally at least one disintegrant and
(f) optionally at least one antioxidant.

According to another preferred embodiment, the granules comprise or essentially consist of
(a) dutasteride,
(b) a solubilizer ,
(c) at least one inert adsorbent,
(d) optionally at least one disintegrant and
(e) optionally at least one antioxidant.

According to another preferred embodiment, the granules comprise or essentially consist of
(a) dutasteride,
(b) a propylene glycol ester,
(c) optionally at least one inert adsorbent,
(d) optionally at least one binder,
(e) optionally at least one disintegrant and
(f) optionally at least one antioxidant.

According to another preferred embodiment, the granules comprise or essentially consist of
(a) dutasteride,
(b) propylene glycol monocaprylate,
(c) optionally at least one inert adsorbent,
(d) optionally at least one binder,
(e) optionally at least one disintegrant and
(f) optionally at least one antioxidant.

According to another preferred embodiment, the granules comprise or essentially consist of
(a) dutasteride,
(b) a solubilizer like a propylene glycol ester such as propylene glycol monocaprylate,
(c) at least one inert adsorbent,
(d) at least one binder,
(e) at least one disintegrant and
(f) optionally at least one antioxidant.

According to another preferred embodiment, the granules comprise or essentially consist of
(a) dutasteride,
(b) a propylene glycol ester such as propylene glycol monocaprylate,
(c) at least one inert adsorbent selected from microcrystalline cellulose, colloidal silicone dioxide and lactose monohydrate,
(d) at least one binder like hydroxypropyl cellulose,
(e) at least one disintegrant like croscarmellose sodium and
(f) optionally at least one antioxidant like butylated hydroxytoluene.

According to another preferred embodiment, the granules comprise or essentially consist of, based on the total weight of the granules,
(a) 0.08 to 0.5 wt.-% of dutasteride,
(b) 5 to 25 wt.-% of a solubilizer like a propylene glycol ester such as propylene glycol monocaprylate,
(c) 50 to 85 wt.-% of at least one inert adsorbent,
(d) 1 to 10 wt.-% of at least one binder,
(e) 1 to 15 wt.-% of at least one disintegrant and
(f) 0 to 0.02 wt.-% of at least one antioxidant.

According to another preferred embodiment, the granules comprise or essentially consist of, based on the total weight of the granules,
(a) 0.08 to 0.5 wt.-% of dutasteride,
(b) 5 to 25 wt.-% of a propylene glycol ester such as propylene glycol monocaprylate,
(c) 50 to 85 wt.-% of at least one inert adsorbent selected from microcrystalline cellulose, colloidal silicone dioxide and lactose monohydrate,
(d) 1 to 10 wt.-% of at least one binder like hydroxypropyl cellulose,
(e) 1 to 15 wt.-% of at least one disintegrant like croscarmellose sodium and
(f) 0 to 0.02 wt.-% of at least one antioxidant.

According to another preferred embodiment, the granules comprise or essentially consist of, based on the total weight of the granules,
(a) 0.08 to 0.5 wt.-% of dutasteride,
(b) 5 to 25 wt.-% of propylene glycol monocaprylate,
(c) 50 to 85 wt.-% of microcrystalline cellulose, colloidal silicone dioxide and lactose monohydrate,
(d) 1 to 10 wt.-% of hydroxypropyl cellulose,
(e) 1 to 15 wt.-% of disintegrant like croscarmellose sodium and
(f) 0 to 0.02 wt.-% of at least one antioxidant.

It has surprisingly been found that the pharmaceutical composition according to the invention is a stable composition showing a suitable dissolution profile of dutasteride and a satisfactory bioavailability of dutasteride. The composition can be prepared by an easy and quick process by simply dissolving dutasteride in a substance having solubilization properties without addition of another surfactant and or co- surfactant. In particular, there is no need to provide a microemulsion comprising dutasteride nor is there a need to micronize dutasteride and, therefore, harmful fine powders of dutasteride can be avoided. Furthermore, there are no special requirements as to the polymorphic form or the particle size of the dutasteride. Excellent content homogeneities can be obtained by the pharmaceutical composition according to the invention.

The composition according to the present invention can be packed into a primary packaging having decreased permeability for water such as high density polyethylene (HDPE) containers with closure, such as polypropylene (PP) closure, and with or without a desiccant; aluminium foil blisters; polychlorotrifluoroethylene (Aclar®) blisters or any other suitable water vapor low permeable packaging such as blisters made of multilayer polymeric foil where different polymeric materials are combined.

The present invention also relates to a stabilized pharmaceutical product comprising a package and packaging method that utilizes an adsorbent material, such as a molecular sieve, that adsorbs moisture in the inner local environment of an impermeable package, so as to prevent hydrolysis and subsequent oxidation products. The adsorbent material can be located in a variety of places. For example, the adsorbent material can be a part of blister foil or can be placed within a porous sachet that, in turn, is located within the sealed package. It is appreciated that numerous adsorbent materials have applications in a stable pharmaceutical product or a method of the present invention, including a molecular sieve, an activated clay, charcoal, an activated alumina, silica, a zeolite, a bauxite, or any mixture of these materials, to name but a few. An effective amount of the adsorbent material used in a stable pharmaceutical product or in a method of the present invention is that an amount sufficient to reduce or eliminate the formation of degradation products. One of ordinary skill can readily determine this amount for a particular embodiment of the present invention using routine laboratory techniques. Moreover, a sealed package of a stable pharmaceutical product or a method of the present invention can be produced from a variety of materials, e.g. metal, glass, plastic, etc. Similarly, the shape of a sealed package can vary. Examples of such shapes include, but certainly are not limited to bottle, a bag, a drum box, and an irregularly shaped container. The sealing of a package of a stable pharmaceutical product or a method of the present invention can be accomplished in a variety of ways. More specifically, heat-sealing, gluing, welding, brazing, mechanical closures, mechanical clamps, or compression can hermetically seal a sealed package of a stable pharmaceutical product of the present invention.

During the packaging and/or storing controlled conditions with low humidity can be used. Additionally inert gases such as nitrogen, argon or helium; or vacuum can be used during the manufacturing of the solid oral dosage forms, the manufacturing of starter, isolated and final pellets including granules and active particles as well as during their packaging to assure inert atmosphere around the solid oral dosage form, such as tablet, pellet or capsule, in the sealed primary packaging. "Inert atmosphere", as used herein, refers to a concentration of oxygen in the atmosphere around the solid dosage form packed in the primary packaging of less than 10 vol%, preferably less than 5 vol% and most preferably less than 2 vol%. The concentration of oxygen in the atmosphere surrounding the pellets, sachets or capsules can be determined by gas chromatography.

The invention also relates to a process for preparing the composition according to the invention comprising
(i) mixing active ingredient (a) with solubilizer (b) to obtain a mixture,
(ii) converting the mixture of step (i) and optionally other excipients into a particulate form to obtain active particles,
(iii) optionally converting the active particles obtained in step (ii) and optionally other excipients to a solid composition,
(iv) optionally coating the composition obtained in step (iii) and
(v) optionally combining the active particles obtained in step (ii), the solid composition obtained in step (iii) or the coated composition obtained in step (iv) with at least one second active ingredient selected from the group consisting of alpha-1-adrenoceptors, antiandrogens and pharmaceutically acceptable salts and solvates thereof or with a composition comprising the same.

In step (i), components (a) and (b) are mixed. This can generally be done by any known process. It is preferred that in step (i) active ingredient (a) is dissolved or dispersed in solubilizer (b) to obtain a solution or dispersion. Steps (i) and/or (ii) of the process according to the invention do particularly not include formation of a microemulsion. If the mixture obtained in step (i) is in the form of a dispersion, then it is preferably not in the form of an emulsion and more preferably not in the form of an emulsion with at least 95% particles having a mean particle size below 200 nm or with an average particle size below 1,000 nm. Thus, if an emulsion is formed in step (i), the average particle size is preferably more than 1,100 nm.

In fact, it is particularly preferred that in step (i) active ingredient (a) is dissolved in solubilizer (b) to obtain a solution. Preferably, step (i) is carried out at a temperature above the melting or softening temperature of solubilizer (b). Thus, active ingredient (a) is preferably dissolved in liquid or molten solubilizer (b).

In step (ii), the mixture of step (i), and in particular the solution of step (i), and optionally other excipients are converted into a particulate form to obtain active particles.

In a first embodiment, the solution obtained in step (i) is mixed with at least one inert adsorbent or a blend of at least one inert absorbent with optionally further excipients whereby the solution of step (i) is adsorbed onto the inert adsorbent(s) or the blend of inert adsorbent(s) and further excipients. Examples of suitable further excipients that can be used according to this process alternative include disintegrants and antioxidants such as those discussed above in connection with the description of the composition according to the invention.

In a second embodiment, the mixture of step (i) is optionally mixed with at least one further excipient and then solidified. For example, a further excipient is dispersed, e.g. suspended, or dissolved in the solution obtained in step (i) and then the obtained dispersion or solution is solidified. Solidification can be effected by e.g. cooling and optionally crushing or milling to obtain granules of appropriate particle size distribution.

In a third embodiment, solidification according to the second embodiment is effected in such a way that pellets having an average particle size in the range of 250 to 1,500pm, preferably 250 to 1,200 µm are obtained. The obtained pellets can be sieved to obtain the desired particle size distribution. Pellets obtained according to this embodiment can preferably be further processed in steps (iii) and/or (iv) to obtain final dosage forms such as tablets or capsules.

It is particularly preferred to carry out step (ii) according to the first embodiment mentioned above.

In any case, at the end of step (ii) active particles, i.e. particles comprising the active ingredient, are obtained.

In step (iii), the obtained active particles and optionally other excipients can be converted into a solid composition. This can be done by any known process such as wet or dry granulation, direct compression methods, granule or pellet filling into hard capsules or combinations thereof.

In a preferred embodiment, the active particles of step (ii) and optionally other excipients like a binder are converted into granules. This can preferably be done by wet granulation, e.g. by granulating the active particles with a binder solution. Examples of suitable binders include those mentioned above in connection with the description of the composition according to the invention. Preferably, the binder and in particular hydroxypropyl cellulose is dissolved in water or ethanol as binder solvent to provide a binder solution for wet granulation of active particles.

Moreover, it is preferred that the obtained granules are further converted into a solid composition. More preferably, the obtained granules are blended with at least one further excipient selected from disintegrants, binders, diluents/fillers, glidants, lubricants and mixtures thereof and then compressed into tablets or filled into capsules. In step (iv), the composition obtained in step (iii) can be coated with a coating material. Tablet coating equipment known from the state of the art can be used for this step. Water, organic solvents such as ethanol, acetone, isopropanol or mixtures thereof can be used for preparing coating dispersions.

Finally, in step (v) the active particles obtained in step (ii), the solid composition obtained in step (iii) or the coated composition obtained in step (iv) can optionally be combined with at least one second active ingredient selected from the group consisting of alpha-1-adrenoceptors, antiandrogens and pharmaceutically acceptable salts and solvates thereof. Preferably, the second active ingredient can be selected from the group consisting of tamsulosin, silodosin, doxazosin, terazosin, alfuzosin, bicalutamide and pharmaceutically acceptable salts and solvates thereof, and more preferably second active ingredient is tamsulosin or a salt thereof.

In a preferred embodiment, a composition of dutasteride according to the present invention is combined with a solid composition containing the second active ingredient. Preferably, both compositions are combined into a solid dosage form, e.g. by filling both compositions into hard capsules made of e.g. gelatin or hypromellose, or by preparing bilayered or inlay tablets. More preferably, dutasteride in the form of film-coated tablets or granules according to the present invention and tamsulosin hydrochloride in the form of pellets, granules or tablets are filled together into a capsule. Most preferably, they are filled into hard gelatine capsules.

In a first alternative of the process according to the invention, in step (i) active ingredient (a) is dissolved in solubilizer (b) and/or binder. Preferably, the obtained solution is then solidified according to the second embodiment of step (ii) described above. The obtained granules can then optionally be sieved and blended with at least one further excipient selected from disintegrants, binders, diluents/fillers, glidants, lubricants and mixtures thereof and then compressed into tablets or filled into capsules.

In a second alternative of the process according to the invention, the pellets obtained according to the third embodiment of step (ii) described above, can be coated in order to prevent direct contact with dutasteride. Film coating is preferably based on water soluble hydrophilic polymers selected form cellulose ethers such as hypromellose, hyprollose, hydroethylcellulose, methyl metacrylates and copolymers of metacrylates and metycrylic acid (also known as Eudragits®), graft copolymer of polyethyleneglycol and polyvinyl alcohol (for example Kollicoat IR®), polyvinyl alcohol, or the like. Optionally other excipients selected form the group of plasticizers, glidants, antiadhesives and pigment are added.

In a third alternative of the process according to the invention, a dispersion, e.g. a suspension or emulsion, or a solution of active ingredient (a) and optionally further excipients selected from diluents, binders, stabilizers and mixtures thereof in an appropriate vehicle, preferably water or a mixture of water and water miscible organic solvents such as ethanol, isopropanol, methanol, is applied to neutral cores such as sugar spheres or pellets made from microcrystalline cellulose by spraying. A dispersion or solution of dutasteride and optionally further excipients in water or water miscible organic solvents can also be applied in a wet granulation process with optionally other excipients. The obtained layered pellets can optionally be further coated by a film coating soluble in water or aqueous solutions to prevent direct exposure or contact of dutasteride to persons handling the product and other active ingredients.

The invention also relates to a pharmaceutical composition obtainable by a process according to the invention.

The invention is also directed to the use of a pharmaceutical composition according to the invention or a combination composition according to the invention for the manufacture of a medicament for the treatment of benign prostatic hyperplasia (BPH).

The invention is now illustrated in more detail by the following examples which are not intended to limit the scope of the claims.

### Examples 1-3: Film coated tablets containing dutasteride

### Example 1

| | **Ingredients** | **Quantity (mg/tablet)** |
|---|---|---|
| 1 | Dutasteride (d₉ₒ < 10µm) | 0.5 |
| 2 | Propylene glycol monocaprylate (Capryol 90) | 20.0 |
| 3 | Microcrystalline cellulose | 68.0 |
| 4 | Colloidal silicone dioxide | 15.0 |
| 5 | Lactose monohydrate | 100.5 |
| 6 | Croscarmellose sodium | 20.0 |
| 7 | Hydroxypropyl cellulose | 16.0 |
| 8 | Purified water | q.s. |
| 9 | Microcrystalline cellulose | 46.4 |
| 10 | Croscarmellose sodium | 10.0 |
| 11 | Magnesium stearate | 3.0 |
| 12 | Colloidal silicone dioxide | 0.6 |
| | **Total cores** | **300.0** |
| 13 | Opadry white 03F58763 | 9.0 |
| | **Total film coated tablets** | **309.0** |

### Example 2

| | **Ingredients** | **Quantity (mg/tablet)** |
|---|---|---|
| 1 | Dutasteride (d₉₀ = 55µm) | 0.5 |
| 2 | Propylene glycol caprylate (Capryol PGMC) | 30.0 |
| 3 | Microcrystalline cellulose | 68.0 |
| 4 | Colloidal silicone dioxide | 15.0 |
| 5 | Lactose monohydrate | 90.5 |
| 6 | Croscarmellose sodium | 20.0 |
| 7 | Hydroxypropyl cellulose | 16.0 |
| 8 | Purified water | q.s. |
| 9 | Microcrystalline cellulose | 46.4 |
| 10 | Croscarmellose sodium | 10.0 |
| 11 | Magnesium stearate | 3.0 |
| 12 | Colloidal silicone dioxide | 0.6 |
| | **Total cores** | **300.0** |
| 13 | Opadry white 03F58763 | 9.0 |
| | **Total film coated tablets** | **309.0** |

### Example 3

| | **Ingredients** | **Quantity (mg/tablet)** |
|---|---|---|
| 1 | Dutasteride | 0.5 |
| 2 | Glyceryl mono- & dicaprylo/ caprate (Capmul MCM) | 40.0 |
| 3 | Microcrystalline cellulose | 68.0 |
| 4 | Colloidal silicone dioxide | 15.0 |
| 5 | Lactose monohydrate | 80.5 |
| 6 | Croscarmellose sodium | 20.0 |
| 7 | Hydroxypropyl cellulose | 16.0 |
| 8 | Purified water | q.s. |
| 9 | Microcrystalline cellulose | 46.4 |
| 10 | Croscarmellose sodium | 10.0 |
| 11 | Magnesium stearate | 3.0 |
| 12 | Colloidal silicone dioxide | 0.6 |
| | **Total cores** | **300.0** |
| 13 | Opadry white 03F58763 | 9.0 |
| | **Total film coated tablets** | **309.0** |

The manufacturing procedure for the compositions according to Examples 1-3 was as follows:
1. Adsorbents (Ingredients No. 3-5) and croscarmellose sodium (Ingredient No. 6) were mixed to obtain a blend.
2. Dutasteride was dissolved in the solubilizer (Ingredient No. 2) to obtain a solution.
3. The solution obtained in step 2 was added to and adsorbed onto the blend of step 1 to obtain active particles.
4. Hydroxypropyl cellulose (Ingredient No. 7) was dissolved in purified water to obtain a binder solution.
5. The active particles from step 3 were granulated with binder solution from step 4 to obtain granules.
6. The granules were dried and mixed with Ingredients No. 9-12 and then compressed into tablets.
7. The obtained tablets were film-coated with Opadry.

### Examples 4-5: Capsules containing dutasteride and tamsulosin

| | **Example 4** | **Example 5** |
|---|---|---|
| | (mg/tablet) | (mg/tablet) |
| **Granules:** | | |
| Tamsulosin hydrochloride | 0.4 | 0.4 |
| Polyethylene oxide | 10.0 | 4.0 |
| Butylated hxdroxytoluene | 0.04 | 0.04 |
| | | |

| **Extragranular Phase:** | | |
|---|---|---|
| Polyethylene oxide | 186.1 | 192.1 |
| Colloidal silicone dioxide | 1.5 | 1.5 |
| Magnesium stearate | 2.0 | 2.0 |

The manufacturing procedure for the compositions according to Examples 4 and 5 was as follows:
1. Tamsulosin hydrochloride and the granular part of polyethylene oxide were granulated with a solution of butylated hxdroxytoluene in ethanol.
2. The obtained granules and the excipients of the extragranular phase were mixed and compressed into tablets.
3. The obtained tamsulosin tablets and dutasteride granules or coated tablets according to Examples 1 to 3 were filled together into capsules having a quantity of 0.5 mg of dutasteride and 0.4 mg of tamsulosin HCl per capsule.

### Example 6: Capsules containing dutasteride and tamsulosin

| **Ingredients** | **Quantity per capsule (mg)** |
|---|---|
| **Pellet core:** | |
| Tamsulosin hydrochloride | 0.400 |
| Microcrystalline cellulose | 138.25 |
| Eudragit L 30 D-55 | 8.250 |
| Triethylcitrate | 0.825 |
| Talc | 8.250 |
| Water (demineralized) | q.s. |

| **Pellet coating:** | |
|---|---|
| Pellet cores | 162.215 |
| Eudragit L 30 D-55 | 10.815 |
| Triethylcitrate | 1.081 |
| Talc | 4.325 |
| Water (demineralized) | q.s. |
| **Total mass of the pellets** | **178.436** |

The manufacturing procedure for the compositions according to Examples 4 and 5 was as follows:
1. Tamsulosin hydrochloride, talc and microcrystalline cellulose were mixed to form a homogeneous powder blend.
2. The powder blend of step 1 was granulated with a suspension of Eudragit and triethyl citrate in water.
3. The granulate obtained in step 2 was extruded and spheronized to form pellets.
4. The pellets of step 3 were dried.
5. The dried pellets were coated with a suspension of Eudragit, triethylcitrate and talc in water to obtain coated tamsulosin pellets.
6. Dutasteride granules or coated tablets according to Examples 1 to 3 and tamsulosin pellets obtained in step 5 were filled together into capsules having a quantity of 0.5 mg of dutasteride and 0.4 mg of tamsulosin HCl per capsule.

### Example 7: Film coated tablets containing dutasteride

| | **Ingredients** | **Quantity (mg/tablet)** |
|---|---|---|
| 1 | Lactose monohydrate | 90.3 |
| 2 | Microcrystalline cellulose | 47.7 |
| 3 | Povidone K-30 | 4.5 |
| 4 | Sodium starch glycolate | 6.0 |
| 5 | Dutasteride | 0.5 |
| 6 | Abs. ethanol | q.s. |
| 7 | Magnesium stearate | 1.0 |
| | **Total cores** | **150.0** |
| 8 | Opadry white 03F58763 | 4.5 |
| | **Total film coated tablets** | **154.5** |

### Example 8: Film coated tablets containing dutasteride

| | **Ingredients** | **Quantity (mg/tablet)** |
|---|---|---|
| 1 | Lactose monohydrate | 90.3 |
| 2 | Microcrystalline cellulose | 47.7 |
| 3 | Povidone K-30 | 4.5 |
| 4 | Sodium starch glycolate | 6.0 |
| 5 | Dutasteride | 0.5 |
| 6 | Isopropyl alcohol | q.s. |
| 7 | Magnesium stearate | 1.0 |
| | **Total cores** | **150.0** |
| 8 | Opadry white 03F58763 | 4.5 |
| | **Total film coated tablets** | **154.5** |

The manufacturing procedure for the compositions according to Examples 7-8 was as follows:
1. Ingredients No. 1-4 were mixed to obtain a blend.
2. Dutasteride was dissolved in ingredient No. 6 (absolute ethanol/isopropyl alcohol) to form a clear solution.
3. The blend obtained in step 1 was granulated using drug solution of step 2.
4. The granules were dried and mixed with magnesium stearate and then compressed into tablets.
7. The obtained tablets were film-coated with Opadry.

## Claims

1. Pharmaceutical composition comprising
(a) at least one active ingredient selected from dutasteride or a pharmaceutically acceptable salt or solvate thereof and
(b) a solubilizer.

2. Pharmaceutical composition according to claim 1, wherein the composition comprises granules comprising the active ingredient (a) and the solubilizer (b).

3. Pharmaceutical composition according to claim 1 or 2, wherein solubilizer (b) has a melting point or softening point of less than about 170°C.

4. Pharmaceutical composition according to any one of claims 1 to 3, wherein solubilizer (b) has an HLB value of 1 to 7.5.

5. Pharmaceutical composition according to any one of claims 1 to 4, wherein solubilizer (b) is selected from the group consisting of propylene glycol esters, medium chain triglycerides, medium chain mono- and diglycerides, long chain mono- and diglycerides, propylene glycol, polyethylene glycols, block copolymers of polyethylene glycol and polypropylene glycol, sucrose fatty acid esters and mixtures thereof.

6. Pharmaceutical composition according to any one of claims 1 to 5, wherein in addition to the solubilizer (b) the composition and/or the granules do not comprise a further surfactant.

7. Pharmaceutical composition according to claim 6, wherein the further surfactant has an HLB value of 8 or more.

8. Pharmaceutical composition according to any one of claims 6 or 7, wherein the further surfactant is selected from the group consisting of polyethylene glycol 400 monostearate, polyoxy 8 stearate, polyethylene glycol 660 hydroxystearate, macrogol 15 hydroxystearate, polyoxyl 10 oleyl ether, oleth 10 PEG monooleyl ether, polysorbate 80, polysorbate 85, polyoxyl 35 castor oil, polyoxyethyleneglycerol triricinoleate 35, polyoxyl-40-hydrogenated castor oil, caprylcaproyl polyoxyglycerides, hydrogenated palm kernel oil PEG-32 esters, lauryl polyoxyglycerides, lauryl macrogol glycerides and polyglyceryl-6-dioleate.

9. Pharmaceutical composition according any one of claims 2 to 8, wherein the granules essentially consist of
(a) at least one active ingredient selected from dutasteride or a pharmaceutically acceptable salt or solvate thereof,
(b) a solubilizer ,
(c) optionally at least one inert adsorbent,
(d) optionally at least one binder,
(e) optionally at least one disintegrant and
(f) optionally at least one antioxidant.

10. Pharmaceutical composition according to any one of claims 1 to 9 further comprising at least one second active ingredient selected from the group consisting of tamsulosin, silodosin, doxazosin, terazosin, alfuzosin, bicalutamide and pharmaceutically acceptable salts and solvates thereof.

11. Pharmaceutical composition according to claim 10, wherein the second active ingredient is tamsulosin or a salt thereof.

12. Process for preparing a pharmaceutical composition according to any one of claims 1 to 11 comprising
(i) mixing active ingredient (a) with solubilizer (b) to obtain a mixture,
(ii) converting the mixture of step (i) and optionally other excipients into a particulate form to obtain active particles,
(iii) optionally converting the active particles obtained in step (ii) and optionally other excipients to a solid composition and
(iv) optionally coating the composition obtained in step (iii) and
(v) optionally combining the active particles obtained in step (ii), the solid composition obtained in step (iii) or the coated composition obtained in step (iv) with at least one second active ingredient selected from the group consisting of tamsulosin, silodosin, doxazosin, terazosin, alfuzosin, bicalutamide and pharmaceutically acceptable salts and solvates thereof or with a composition comprising the same.

13. Process according to claim 12, wherein in step (i) active ingredient (a) is dissolved in solubilizer (b) to obtain a solution.

14. Process according to claim 13, wherein in step (ii) the solution obtained in step (i) is mixed with at least one inert adsorbent or a blend of at least one inert absorbent with optionally further excipients.

15. Pharmaceutical composition obtainable according to a process according to any one of claims 12 to 14.
